# EUROPEAN PATENT SPECIFICATION

(11) **EP 1 827 579 B1**
(45) Date of publication and mention of the grant of the patent: **15.08.2012**
(21) Application number: 05825833.6
(22) Date of filing: 16.11.2005
(51) Int. Cl.: A61N 1/05

(54) **DIAPHRAGMATIC PACING WITH PHYSIOLOGICAL NEED ADJUSTMENT**
DIAPHRAGMA-PACING MIT PHYSIOLOGISCHER BEDARFSANPASSUNG
ELECTROSTIMULATION DU DIAPHRAGME AVEC REGLAGE DES BESOINS PHYSIOLOGIQUES

(30) Priority: 03.12.2004 US 4384
(43) Date of publication of application: 05.09.2007
(73) Proprietor: The Alfred E. Mann Foundation for Scientific Research, Santa Clarita, CA 91380-9005 (US)
(72) Inventor: MANDELL, Lee, J., West Hills, CA 91304-6011 (US)
(74) Representative: Carter, Stephen John
(86) International application number: PCT/US2005/041485
(87) International publication number: WO 2006/062710

(56) References cited:
- EP-A- 1 393 773
- US-A- 5 146 918
- US-A- 5 370 667
- US-A- 5 524 632
- US-A- 5 810 735
- US-A- 6 164 284
- US-A- 6 164 284
- US-A1- 2003 153 953
- US-A1- 2005 021 102
- US-A1- 2005 085 866
- US-A1- 2005 085 869
- US-B1- 6 415 183
- US-B1- 6 415 183
- US-B2- 6 811 538
- US-B2- 6 811 538

## Description

### Field of the Invention

The present invention is generally directed to systems for providing diaphragmatic pacing to a patient having an associated neurological deficit and is particularly applicable to patients who are or can be re-animated, e.g., via a system of discrete implantable medical devices, and thus need their breathing to be responsive to variations in their physiological need.

### Background of the Invention

Diaphragm Pacing Stimulation (DPS) for ventilator-dependent patients provides several advantages over conventional techniques such as phrenic nerve pacing or mechanical ventilator support. It avoids potential phrenic nerve injury as well as high cost hospitalization. Synapse Biomedical (Oberlin, OH) is commercializing a device with intramuscular electrodes that are implanted laparoscopically into the diaphragm near the phrenic nerve motor points. At the time of writing, over nine patients have been implanted with this leaded system attached percutaneously to an external NeuRx RA/4 Pulse Generator (PG). The results of the clinical trials with these patients are encouraging. The known patients are quadriplegics and require DPS or a ventilator, due to spinal cord injuries, e.g., a C2 spinal cord injury, that resulted in the paralysis and also the neurological deficit corresponding to the need for ventilation support. The assignee of the present invention has developed a device or, more specifically, a system of devices that provides the promise to restore motor movements to such patients. For example, see U.S. Pat. Nos. 6,164,284; 6,185,452; 6,208,894; 6,315,721; 6,564,807; and their progeny, each of which is incorporated herein by reference in its entirety. Such a device may be referred to as a BION^{®}, a trademark of Advanced Bionics Corporation. However, when this promise is fulfilled, current DPS systems (which cause respiration to occur at a fixed rate) will be inadequate since they cannot respond to the patient's physiological need, e.g., cardiovascular demand levels, without manual intervention.

US Patent Application, US 6415183, shows a diaphragmatic pacing system and method wherein an electrical stimulis is delivered to the phrenic nerve when a signal indicates that the respiratory activity is below a predetermined level.

European Patent Application, EP1 393 773, shows a nerve stimulation device which also stimulates the phrenic nerve.

US Patent Application, US 2003/015 3953, shows an implantable cardiac stimulation device which can generate cardiac pacing pulses to prevent a sleep apnea condition.

US Patent Application, US 6811538, shows a technique for collecting and analysing physiological signals to detect sleep apnea.

US Patent Application, US 61 64 284, shows a system for monitoring and/or affecting parameters of a patient's body wherein the system is implanted in the patient. Wireless communication within the system is possible.

### Summary of the Invention

The present invention is defined in the appended claims. The embodiments of the description which do not fall within the scope of the claims are provided for illustrative purposes and do not from part of the invention. The present invention is directed to a system that provides adjustable diaphragmatic pacing to a patient having an associated neurological deficit wherein the adjustments automatically occur In response to the patient's physiological need. In a first Implementation, physiological need is determined according to the patient's activity level, e.g., as determined by the patient's motion as detected by one or more accelerometers. In a second implementation, physiological need is determined by an oximeter measuring the current oxygen level of the patient's blood. In a third implementation, physiological need is determined by a combination of the motion sensed by an accelerometer and further adjusted by the oxygen level sensed by an oximeter. Preferably, systems of the present invention are Implantable and powered by rechargeable batteries and may be integrated with a system of Implantable devices that restores motor functions to an injured patient and this restoration then requires an adjustable respiration rate in response to the patient's restored movements.

In an exemplary environment, a system control unit (SCU) comprises a programmable unit capable of (1) transmitting commands to at least some of a plurality of implanted devices and (2) receiving data signals from at least some of those implanted devices. Preferably, the system operates in a closed loop fashion whereby the commands transmitted by the SCU are dependent, in part, on the content of the data signals received by the SCU. Such systems hold the promise of restoring motor functions to paraplegics, e.g., quadriplegics.

Implanted devices in this exemplary environment may be configured similarly to the devices described In the commonly owned U.S. Pat. No. 6,164,284 (hereinafter referred to as the'284 patent), are typically contained within a sealed housing suitable for injection into the patient's body. Each housing preferably contains a power source having a capacity of at least 1 microwatt-hour and power consuming circuitry preferably including a data signal transmitter and receiver and sensor/stimulator circuitry for driving an input/output transducer. Wireless communication between the SCU and the other implanted devices can be implemented in various ways, e.g., via a modulated sound signal, an AC magnetic field, an RF signal, a propagated electromagnetic wave, a light signal, or electrical conduction. Furthermore, in commonly owned U.S. Pat. No. 6,472,991 entitled Multichannel Communication Protocol Configured to Extend The Battery Life Of An Implantable Device", incorporated herein by reference in its entirety, a communication protocol is described for an exemplary communication protocol for communicating between a master device (also referred to herein and In the associated patents as a system control unit (SCU)) which may be implanted within or in proximity to a patient that communicates with a plurality of discrete implantable slave devices, suitable for implantation via injection, via a wireless communication channel.

Alternatively, implanted devices In this exemplary environment may be configured similarly to the devices described in the commonly owned U.S. Pat. Nos. 5,193,539 and 5,193,540 (herein referred to as the '539 and '540 patents). Such devices differ from those devices described in the '284 patent in that they do not contain a battery and instead rely upon an externally-provided AC magnetic field to Induce a voltage, e.g., via a coil into an internal capacitor, and thus power its internal electronics only when the external AC magnetic field Is present. These devices are also referred to as being RF powered. Systems which comprise the environment of the present invention may include either the '284 battery-powered or the '539/'540 RF-powered classes of devices or combinations thereof.

In accordance with the present invention, a preferred system for controlling respiration in a patient having an associated neurological deficit by using an electronic device configured for adjustably diaphragmatically pacing in response to the patient's physiological need comprises (1) one or more pulse generators configured for delivering stimulation pulses and coupling to a plurality of electrodes and suitable for placement proximate to a neurological pathway that responds to said stimulation pulses to induce respiration, wherein the one or more pulse generators operate at a stimulation rate; (2) a sensor for measuring a parameter corresponding to the patient's physiological need; and wherein the stimulation rate of the one or more pulse generators is periodically adjusted according to the parameter measured by the sensor.

In a further aspect of embodiments of the present invention, the parameter corresponding to the patient's need is determined by a sensor for measuring activity level, e.g., by one or more accelerometers. Alternatively, the patient's physiological need is determined by measuring the patient's blood oxygen level using an oximeter.

In a still further aspect of embodiments of the present invention, the system is suitable for implantation and may be contained within a sealed elongate housing having an axial dimension of less than 60 mm and a lateral dimension of less than 6 mm. Such implantable devices receive power from an externally-provided magnetic field and may contain a rechargeable battery to maintain its function during periods of time when the externally-provided magnetic field is no longer present.

Finally, embodiments of the present invention may be a portion of a system of devices, e.g., a master controller and one or more implantable slave devices, that may be used for restoring other motor functions to a patient having additional motor deficits.

The novel features of the invention are set forth with particularity in the appended claims. The invention will be best understood from the following description when read in conjunction with the accompanying drawings.

### Brief Description of the Drawings

**FIG. 1** is a simplified block diagram of an exemplary system suitable for forming an exemplary environment for the use of the present invention, the system being comprised of implanted devices, e.g., microstimulators, microsensors and microtransponders, under control of a system control unit (SCU).
**FIG. 2** comprises a block diagram of the system of **FIG. 1** showing the functional elements that form the system control unit and implanted microstimulators, microsensors and microtransponders.
**FIG. 3A** comprises a block diagram of an exemplary implantable device, as shown in U.S. Pat. No. 6,164,284, including a battery for powering the device for a period of time in excess of one hour in response to a command from the system control unit.
**FIG. 3B** comprises a simplified block diagram of controller circuitry that can be substituted for the controller circuitry of **FIG. 3A**, thus permitting a single device to be configured as a system control unit and/or a microstimulator and/or a microsensor and/or a microtransponder.
**FIG. 4** shows an exemplary flow chart of the use of an exemplary system in an open loop mode for controlling/monitoring a plurality of implanted devices, e.g., microstimulators, microsensors.
**FIG. 5** shows a simplified flow chart of the use of closed loop control of a microstimulator by altering commands from the system control unit in response to status data received from a microsensor.
**FIG. 6** shows an exemplary injury, i.e., a damaged nerve, and the placement of a plurality of implanted devices, i.e., microstimulators, microsensors and a microtransponder under control of the system control unit for "replacing" the damaged nerve.
**FIG. 7** shows a simplified flow chart of the control of the implanted devices of **FIG. 6** by the system control unit.
**FIG. 8** shows a simplified flow chart of the process of adjusting the diaphragmatic pacing rate provided by embodiments of the present invention in response to one or more measured physiological parameters, e.g., movement as sensed by an accelerometer, blood oxygen level and/or pulse rate as measured by a pulse oximeter.
**FIG. 9** shows a simplified block diagram of a first set of embodiments of the present invention in which a pair of implantable stimulators are used to provide stimulation to the right and left hemidiaphragm motor points at a rate responsive to measurements corresponding to the patient's current physiological need.
**FIGS. 10A-10C** show simplified block diagrams of a second set of embodiments of the present invention in which a single implantable stimulator is used to provide stimulation to the right and left hemidiaphragm motor points at a rate responsive to measurements corresponding to the patient's current physiological need.
**FIG. 11** shows a simplified block diagram of a third set of embodiments of the present invention in which an external stimulator is used to provide stimulation to the right and left hemidiaphragm motor points via a percutaneous connection at a rate responsive to measurements corresponding to the patient's current physiological need.
**FIG. 12** shows a simplified block diagram of a fourth set of embodiments of the present invention in which a single implantable stimulator is used to provide stimulation to the right and left hemidiaphragm motor points via stimulation of the phrenic nerves at a rate responsive to measurements corresponding to the patient's current physiological need. Alternatively, an embodiment is shown where a stimulator having an integrated nerve cuff is coupled to each of the phrenic nerves.

### Detailed Description of the Preferred Embodiments

The following description is of the best mode presently contemplated for carrying out the invention. This description is not to be taken in a limiting sense, but is made merely for the purpose of describing the general principles of the invention. The scope of the invention is defined by the appended claims.

The present invention is directed to a system that provides adjustable diaphragmatic pacing to a patient having an associated neurological deficit wherein the adjustments automatically occur in response to the patient's physiological need. In a first implementation, physiological need is determined according to the patient's activity level, e.g., as determined by the patient's motion as detected by one or more accelerometers. In a second implementation, physiological need is determined by an oximeter measuring the current oxygen level of the patient's blood or the patient's pulse rate. Preferably, systems of the present invention are implantable and powered by rechargeable batteries and may be integrated with a system of implantable devices that restores motor functions to an injured patient and this restoration then requires an adjustable respiration rate in response to the patient's restored movements.

In an exemplary environment, the SCU (a master device) comprises a programmable unit capable of transmitting commands to at least some of a plurality of implanted devices (slave devices) and may also be capable of receiving data signals from at least some of those implanted devices. Preferably, the system operates, at least in part, in a closed loop fashion whereby the commands transmitted by the SCU are dependent, in part, on the content of the data signals received by the SCU.

Each implanted device in this exemplary environment is configured similarly to the devices described in the commonly owned U,S. Pat. No. 6,164,284 (hereinafter referred to as the '284 patent) and are typically contained within a sealed housing suitable for injection into the patient's body. Each housing preferably contains a power source having a capacity of at least 1 microwatt-hour, preferably a rechargeable battery, and power consuming circuitry preferably including a data signal transmitter and receiver and sensor/stilmulator circultry for driving an input/output transducer.

Alternatively, implanted devices In this exemplary environment may be configured similarly to the devices described in the commonly owned U.S. Pat. Nos. 5,193,539 and 5,193,540, herein referred to as the '539 and '540 patents. Such devices differ from those devices described in the '284 patent In that they do not contain a battery and instead rely upon an externally-provided alternating magnetic field to induce a voltage, e.g., via a coil into an internal capacitor, and thus power its internal electronics only when the external alternating magnetic field is present. These devices are also referred to as being RF powered. Systems which comprise an exemplary environment for use of the present invention may include either the '284 battery-powered or '539/'540 RF-powered classes of devices or combinations thereof.

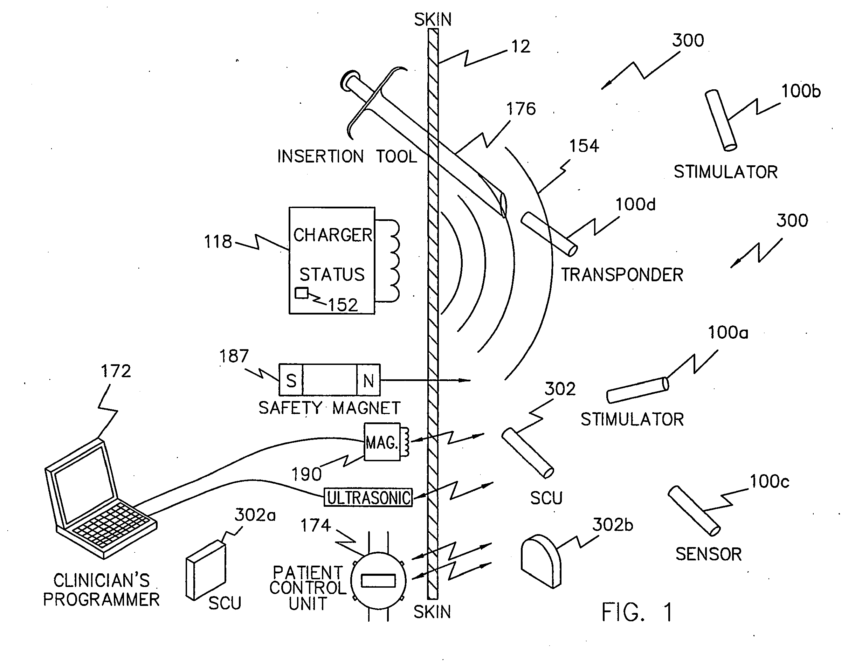

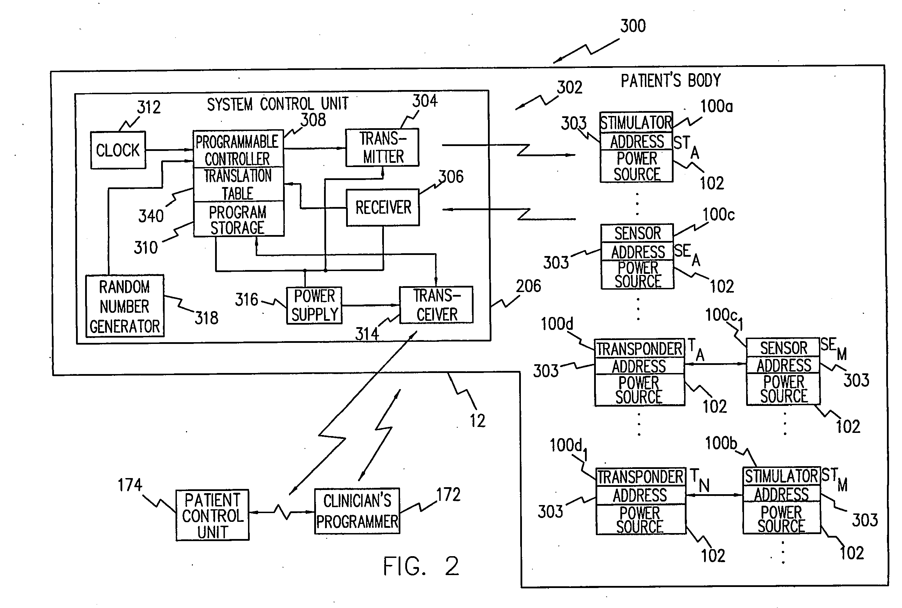

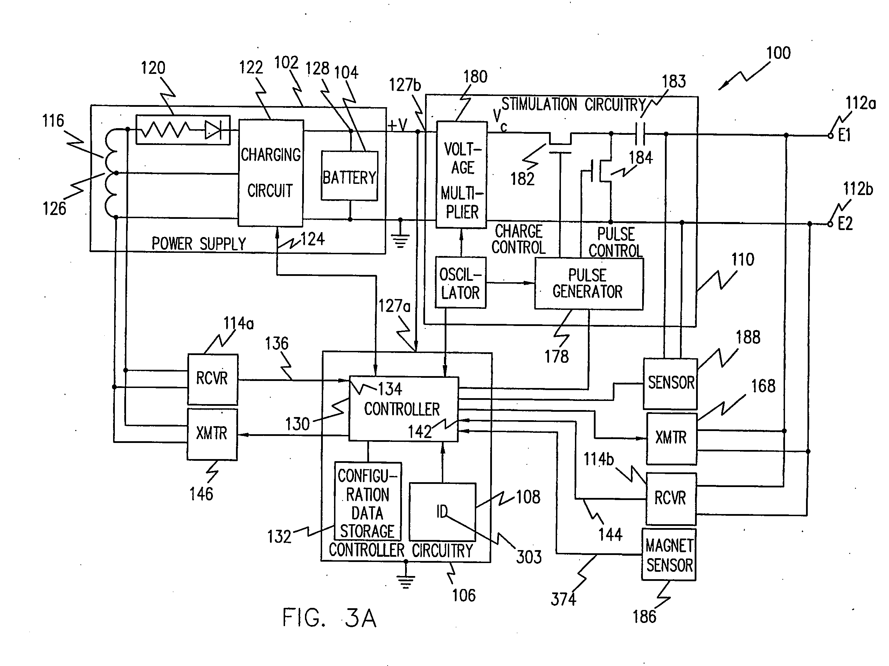

**FIGS. 1** **and** **2** show an exemplary system 300 made of implanted devices 100, preferably battery powered, under control of a system control unit (SCU) 302, preferably also implanted beneath a patient's skin 12. As described in the '284 patent, potential implanted devices 100 (see also the block diagram shown in FIG. 3A) include stimulators, e.g., 100a and 100b, sensors, e.g., 100c, and transponders, e.g., 100d. The stimulators, e.g., 100a, can be remotely programmed to output a sequence of drive pulses to body tissue proximate to its implanted location via attached electrodes. The sensors, e.g., 100c, can be remotely programmed to sense one or more physiological or biological parameters In the Implanted environment of the device, e.g., temperature, glucose level, O₂ content, nerve potential, muscle potential, etc. Transponders, e.g., 100d, are devices which can be used to extend the interbody communication range between stimulators and sensors and other devices, e.g., a clinician's programmer 172 and the patient control unit 174. Preferably, these stimulators, sensors and transponders are contained in sealed elongate housings having an axial dimension of less than 60 mm and a lateral dimension of less than 6 mm. Accordingly, such stimulators, sensors and transponders are respectively referred to as microstimulators, microsensors, and microtransponders or referred to in general as battery-powered, implantable stimulator/sensor devices. Such microstimulators and microsensors can thus be positioned beneath the skin 12 within a patient's body using a hypodermic type insertion tool 176.

As described in the '284 patent, microstimulators and microsensors are remotely programmed and interrogated via a wireless communication channel, e.g., modulated AC magnetic, sound (i.e., ultrasonic), RF or electric fields, typically originating from control devices external to the patient's body, e.g., the clinician's programmer 172 or patient control unit 174. Typically, the clinician's programmer 172 is used to program a single continuous or one time pulse sequence into each microstimulator and/or measure a biological parameter from one or more microsensors. Similarly, the patient control unit 174 typically communicates with the implanted devices 100, e.g., microsensors 100c, to monitor biological parameters. In order to distinguish each implanted device over the communication channel, each implanted device is manufactured with a unique address or identification code (ID) 303 specified in address storage circuitry 108 (see **FIG. 3A**) as described in the '284 patent. Unique is a relative term, e.g., the more bits used to specify the identification code the easier it will be to distinguish one device or, in the case of master devices, one system of devices from another system of devices. Accordingly, as used in this patent application, unique is only intended to specify that the ID 303 is distinguishable from the IDs of other devices that may exist within the same environment.

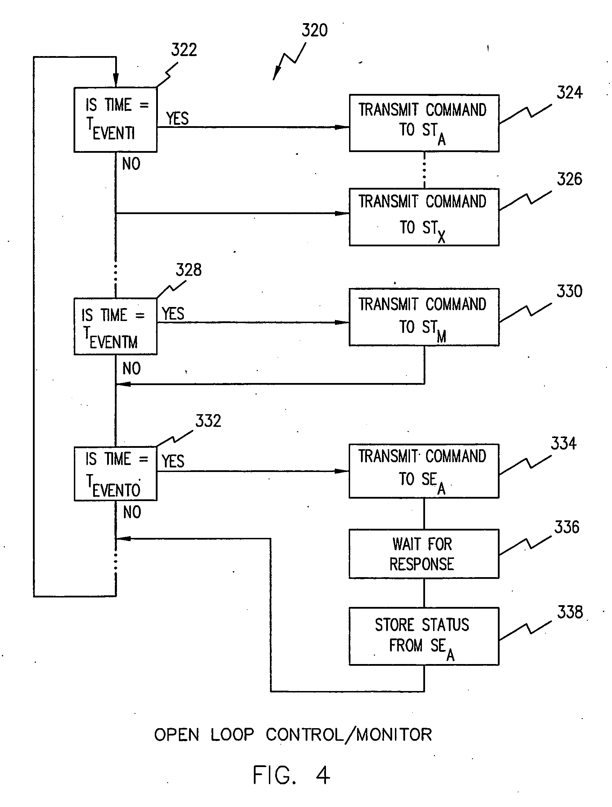

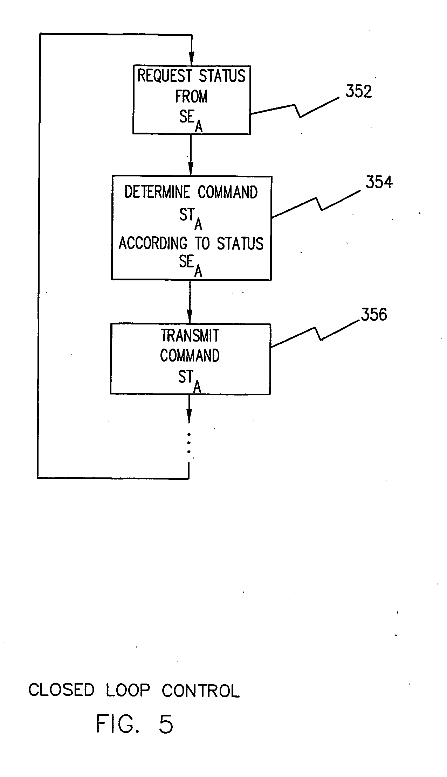

By using one or more such implantable devices in conjunction with the SCU 302, the capabilities of such implanted devices can be further expanded. For example, in an open loop mode (described below in reference to **FIG. 4**), the SCU 302 can be programmed to periodically initiate tasks, e.g., perform real time tasking, such as transmitting commands to microstimulators according to a prescribed treatment regimen or periodically monitor biological parameters to determine a patient's status or the effectiveness of a treatment regimen. Alternatively, in a closed loop mode (described below in reference to **FIGS. 5-7**), the SCU 302 may periodically interrogate one or more microsensors and accordingly adjust the commands transmitted to one or more microstimulators.

**FIG. 2** shows an exemplary system 300 comprised of (1) one or more implantable devices 100 operable to sense and/or stimulate a patient's body parameter in accordance with one or more controllable operating parameters and (2) the SCU 302. The SCU 302 is primarily comprised of (1) a housing 206, preferably sealed and configured for implantation beneath the skin of the patient's body, e.g., as described in the '284 patent in reference to the implanted devices 100, (2) a signal transmitter 304 in the housing 206 for transmitting command signals, (3) a signal receiver 306 in the housing 206 for receiving status signals, and (4) a programmable controller 308, e.g., a microcontroller or state machine, in the housing 206 responsive to received status signals for producing command signals for transmission by the signal transmitter 304 to other implantable devices 100. The sequence of operations of the programmable controller 308 is determined by an instruction list, i.e., a program, stored in program storage 310, coupled to the programmable controller 308. While the program storage 310 can be a nonvolatile memory device, e.g., ROM, manufactured with a program corresponding to a prescribed treatment regimen, it is preferable that at least a portion of the program storage 310 be an alterable form of memory, e.g., RAM, EEPROM, etc., whose contents can be remotely altered as described further below. However, it is additionally preferable that a portion of the program storage 310 be nonvolatile so that a default program is always present. The rate at which the program contained within the program storage 310 is executed is determined by clock/oscillator 312. Additionally, a real time clock operating in response to clock/oscillator 312 preferably permits tasks to be scheduled at specified times of day.

The signal transmitter 304 and signal receiver 306 preferably communicate with implanted devices 100 using an RF signal, e.g., a propagated electromagnetic wave, modulated by a command data signal. Alternatively, an audio transducer may be used to generate mechanical vibrations having a carrier frequency modulated by a command data signal. In an exemplary embodiment, a carrier frequency of 100 kHz is used which corresponds to a frequency that freely passes through a typical body's fluids and tissues. However, such sound means that operate at any frequency, e.g., greater than 1 Hz, are also considered to be suitable for a potential communication channel. Alternatively, the signal transmitter 304 and signal receiver 306 can communicate using modulated AC magnetic fields.

The clinician's programmer 172 and/or the patient control unit 174 and/or other external control devices can also communicate with the implanted devices 100, as described in the '284 patent, preferably using a modulated RF or AC magnetic field. Alternatively, such external devices can communicate with the SCU 302 via a transceiver 314 coupled to the programmable controller 308. Since, the signal transmitter 304 and signal receiver 306 may operate using a different communication means, a separate transceiver 314 which operates using an alternative communication means may be used for communicating with external devices. However, a single transmitter 304/receiver 306 can be used in place of transceiver 314 for communicating with the external devices and implanted devices if a common communication channel is used.

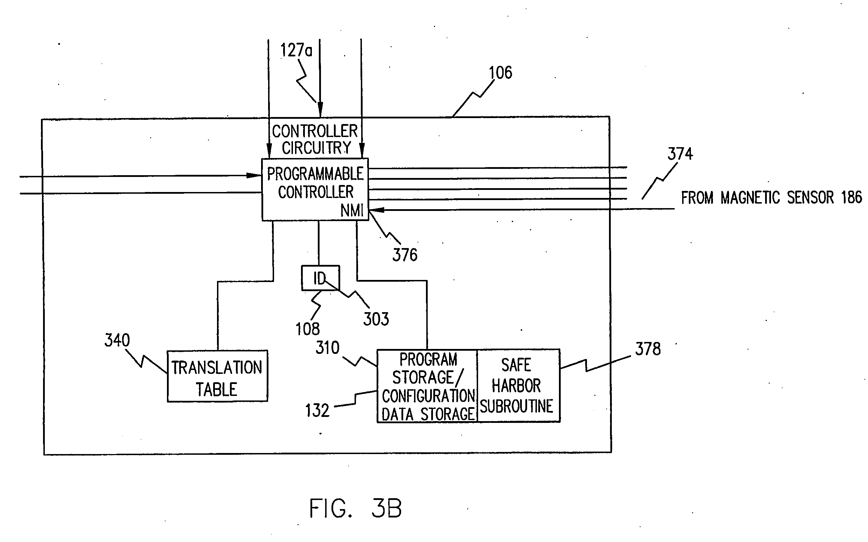

**FIG. 3A** comprises a block diagram of an exemplary implantable device 100 operable under control of controller circuitry 106 and includes a battery 104, preferably rechargeable, for powering the device for a period of time in excess of one hour and responsive to command signals from a remote master device, e.g., the SCU 302. The controller circuitry 106 is primarily comprised of a controller 130, configuration data storage 132 for prescribing its operation, and address storage circuitry 108 for storing the ID 303 of the device. As described in the '284 patent, the implantable device 100 is preferably configurable to alternatively operate as a microstimulator and/or microsensor and/or microtransponder due to the commonality of most of the circuitry contained within. Such circuitry may be further expanded to permit a common block of circuitry to also perform the functions required for the SCU 302. Accordingly, **FIG. 3B** shows an alternative implementation of the controller circuitry 106 of **FIG. 3A** that is suitable for implementing a microstimulator and/or a microsensor and/or a microtransponder and/or the SCU 302. In this implementation, the configuration data storage 132 can be alternatively used as the program storage 310 when the implantable device 100 is used as the SCU 302. In this implementation, XMTR 168 corresponds to the signal transmitter 304 and the RCVR 114b corresponds to the signal receiver 306 (preferably operable via electrodes 112a and 112b operating as an RF antenna) and the RCVR 114a and XMTR 146 correspond to the transceiver 314 (preferably operable via coil 116 for AC magnetic modes of communication).

Preferably, the contents of the program storage 310, i.e., the software that controls the operation of the programmable controller 308, can be remotely downloaded, e.g., from the clinician's programmer 172 using data modulated onto an RF signal or an AC magnetic field. In this mode, it is preferable that the contents of the program storage 310 for each SCU 302 be protected from an inadvertent change. Accordingly, the contents of the address storage circuitry 108, i.e., the ID 303, is preferably used as a security code to confirm that the new program storage contents are destined for the SCU 302 receiving the data. This feature is particularly significant if multiple patients could be physically located, e.g., in adjoining beds, within the communication range of the clinician's programmer 172.

Preferably, the SCU 302 can operate for an extended period of time, e.g., in excess of one hour, from an internal power supply 316 (see **FIG. 2**). While a primary battery, i.e., a nonrechargeable battery, is suitable for this function, it is preferable that the power supply 316 include a rechargeable battery, e.g., battery 104 as described in the '284 patent, that can be recharged via an AC magnetic field produced external to the patient's body. Accordingly, power supply 102 of **FIG. 3A** is the preferred power supply 316 for the SCU 302 as well.

The battery-powered devices 100 of the '284 patent are preferably configurable to operate in a plurality of operational modes, e.g., via a communicated command signal. In a first operational mode, device 100 is remotely configured to be a microstimulator, e.g., 100a and 100b. In this embodiment (see **FIG. 3A**), controller 130 commands stimulation circuitry 110 to generate a sequence of drive pulses through electrodes 112 to stimulate tissue, e.g., a nerve or muscle, proximate to the implanted location of the microstimulator, e.g., 100a or 100b. In operation, a programmable pulse generator 178 and voltage multiplier 180 are configured with parameters corresponding to a desired pulse sequence and specifying how much to multiply (or divide) the battery voltage (e.g., by summing charged capacitors or similarly charged battery portions) to generate a desired compliance voltage V_{c}. A first FET 182 is periodically energized to store charge into capacitor 183 (in a first direction at a low current flow rate through the body tissue) and a second FET 184 is periodically energized to discharge capacitor 183 in an opposing direction at a higher current flow rate which stimulates a nearby muscle or nerve. Alternatively, electrodes can be selected that will form an equivalent capacitor within the body tissue.

In a next operational mode, the battery-powered implantable device 100 can be configured to operate as a microsensor, e.g., 100c, that can sense one or more physiological or biological parameters in the implanted environment of the device. In accordance with a preferred mode of operation, the system control unit 302 periodically requests the sensed data from each microsensor 100c using its ID 303 stored in the address storage circuitry 108, and responsively sends command signals to microstimulators, e.g., 100a and 100b, adjusted according to the sensed data. For example, sensor circuitry 188 can be coupled to the electrodes 112 to sense or otherwise used to measure a biological parameter, e.g., temperature, glucose level, O₂ content, voltage, current, impedance, etc., and provide the sensed data to the controller circuitry 106. Preferably, the sensor circuitry 188 includes a programmable bandpass filter and an analog to digital (A/D) converter that can sense and accordingly convert the voltage levels across the electrodes 112 into a digital quantity. Alternatively, the sensor circuitry 188 can include one or more sense amplifiers to determine if the measured voltage exceeds a threshold voltage value or is within a specified voltage range. Furthermore, the sensor circuitry 188 can be configurable to include integration circuitry to further process the sensed voltage. The operational mode of the voltage sensor circuitry 188 is remotely programmable via the device's communication interface.

Additionally, the sensing capabilities of a microsensor preferably include the capability to monitor the battery status via path 124 from the charging circuit 122 and can additionally include using an ultrasonic transducer, i.e., emitter/receiver (not shown), or the coil 116 to respectively measure the ultrasonic, magnetic, or propagated RF signal magnitudes (or communication time delays) of signals transmitted between a pair of implanted devices and thus determine the relative locations of these devices. This information can be used to determine the amount of body movement, e.g., the amount that an elbow or finger is bent, and thus form a portion of a closed loop motion control system.

In another operational mode, the battery-powered implantable device 100 can be configured to operate as a microtransponder, e.g., 100d. In this operational mode, the microtransponder receives (via the aforementioned RCVR 114a using AC magnetic, sonic, RF, or electric communication modes) a first command signal from the SCU 302 and retransmits this signal (preferably after reformatting) to other implanted devices (e.g., microstimulators, microsensors, and/or microtransponders) using the aforementioned XMTR 168 using magnetic, sonic, RF, or electric communication modes. While a microtransponder may receive one mode of command signal, e.g., magnetic, it may retransmit the signal in another mode, e.g., RF. For example, clinician's programmer 172 may emit a modulated magnetic signal using a magnetic emitter 190 (see **FIG. 1**) to program/command the implanted devices 100. However, the magnitude of the emitted signal may not be sufficient to be successfully received by all of the implanted devices 100. As such, a microtransponder 100d may receive the modulated magnetic signal and retransmit it (preferably after reformatting) as a modulated ultrasonic or RF signal which can pass through the body with fewer restrictions. In another exemplary use, the patient control unit 174 may need to monitor a microsensor 100c in a patient's foot. Despite the efficiency of ultrasonic, magnetic, and propagated RF communication in a patient's body, such a signal could still be insufficient to pass from a patient's foot to a patient's wrist (the typical location of the patient control unit 174). As such, a microtransponder 100d could be implanted (if needed) in the patient's torso to improve the communication link.

**FIG. 4** shows a block diagram of an exemplary open loop control program, i.e., a task scheduler 320, for controlling/monitoring a body function/parameter. In this process, the programmable controller 308 is responsive to the clock 312 (preferably a crystal controlled oscillator to thus permit real time scheduling) in determining when to perform any of a plurality of tasks. In this exemplary flow chart, the programmable controller 308 first determines in block 322 if it is now at a time designated as T_{EVENT1} (or at least within a sampling error of that time), e.g., at 1:00 AM. If so, the programmable controller 308 transmits a designated command to microstimulator A (ST_{A}) in block 324. In this example, the control program continues where commands are sent to a plurality of stimulators and concludes in block 326 where a designated command is sent to microstimulator X (ST_{X}). Such a subprocess, e.g., a subroutine, is typically used when multiple portions of body tissue require stimulation, e.g., stimulating a plurality of muscle groups in a paralyzed limb to avoid atrophy. The task scheduler 320 continues through multiple time event detection blocks until in block 328, it determines whether the time T_{EVENTM} has arrived. If so, the process continues at block 330 where, in this case, a single command is sent to microstimulator M (ST_{M}). Similarly, in block 332, the task scheduler 320 determines when it is the scheduled time, i.e., T_{EVENTO}, to execute a status request from microsensor A (SE_{A}). If so, a subprocess, e.g., a subroutine, commences at block 334 where a command is sent to microsensor A (SE_{A}) to request sensor data and/or specify sensing criteria. Microsensor A (SE_{A}) does not instantaneously respond. Accordingly, the programmable controller 308 waits for a response in block 336. In block 338, the returned sensor status data from microsensor A (SE_{A}) is stored in a portion of the memory, e.g., a volatile portion of the program storage 310, of the programmable controller 308. The task scheduler 320 can be a programmed sequence, i.e., defined in software stored in the program storage 310, or, alternatively, a predefined function controlled by a table of parameters similarly stored in the program storage 310. A similar process may be used where the SCU 302 periodically interrogates each implantable device 100 to determine its battery status.

**FIG. 5** is an exemplary block diagram showing the use of such a system to perform closed loop control of a body function. In block 352, the SCU 302 requests status from microsensor A (SE_{A}). The SCU 302, in block 354, then determines whether the present command given to a microstimulator is satisfactory and, if necessary, determines a new command and transmits the new command to the microstimulator A (ST_{A}) in block 356. For example, if microsensor A (SE_{A}) is reading a voltage corresponding to the degree of contraction resulting from stimulating a muscle, the SCU 302 could transmit a command to microstimulator A (ST_{A}) to adjust the sequence of drive pulses, e.g., in magnitude, duty cycle, etc., and accordingly change the voltage sensed by microsensor A (SE_{A}). Accordingly, closed loop, i.e., feedback, control is accomplished. The characteristics of the feedback (proportional, integral, derivative (PID)) control are preferably program controlled by the SCU 302 according to the control program contained in program storage 310.

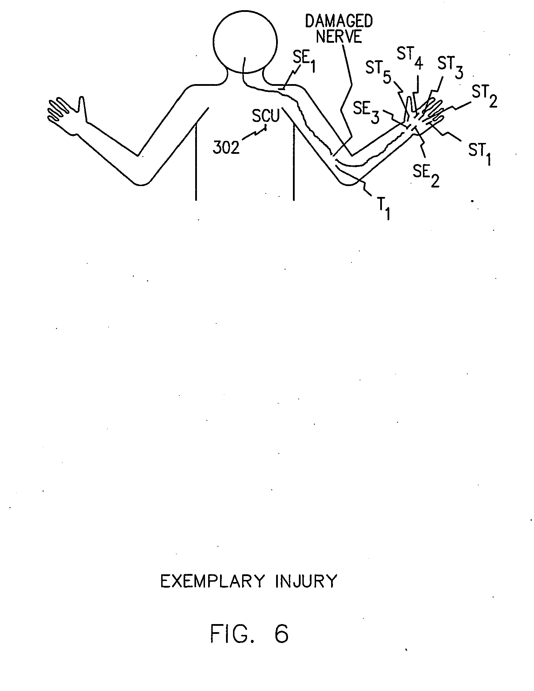

**FIG. 6** shows an exemplary injury treatable by such an exemplary system 300. In this exemplary injury, the neural pathway has been damaged, e.g., physically or effectively (as a consequence of a stroke or the like) severed, just above the patient's left elbow. The goal of this exemplary system is to bypass the damaged neural pathway to permit the patient to regain control of the left hand. An SCU 302 is implanted within the patient's torso to control a plurality of stimulators, ST₁-ST₅, implanted proximate to the muscles respectively controlling the patient's thumb and fingers (shown in the patient's hand for simplicity). Additionally, microsensor 1 (SE₁) is implanted proximate to an undamaged nerve portion where it can sense a signal generated from the patient's brain when the patient wants hand closure. Optional microsensor 2 (SE₂) is implanted in a portion of the patient's hand where it can sense a signal corresponding to stimulation/motion of the patient's pinky finger and microsensor 3 (SE₃) is implanted and configured to measure a signal corresponding to grip pressure generated when the fingers of the patient's hand are closed. Additionally, an optional microtransponder (T₁) is shown which can be used to improve the communication between the SCU 302 and the implanted devices.

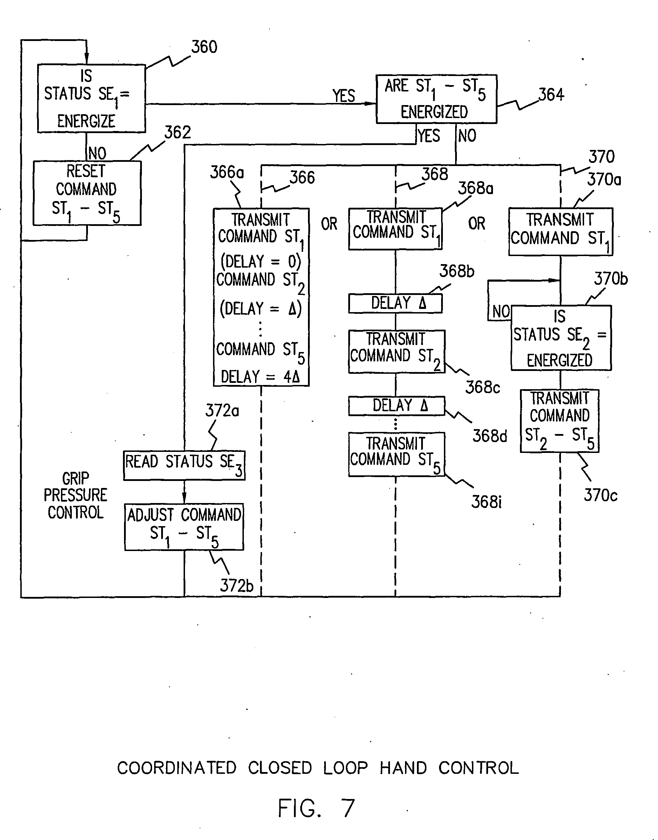

**FIG. 7** shows an exemplary flow chart for the operation of the SCU 302 in association with the implanted devices in the exemplary system of **FIG. 6****.** In block 360, the SCU 302 interrogates microsensor 1 (SE₁) to determine if the patient is requesting actuation of his fingers. If not, a command is transmitted in block 362 to all of the stimulators (ST₁-ST₅) to open the patient's hand, i.e., to de-energize the muscles which close the patient's fingers. If microsensor 1 (SE₁) senses a signal to actuate the patient's fingers, the SCU 302 determines in block 364 whether the stimulators ST₁-ST₅ are currently energized, i.e., generating a sequence of drive/stimulation pulses. If not, the SCU 302 executes instructions to energize the stimulators. In a first optional path 366, each of the stimulators is simultaneously (subject to formatting and transmission delays) commanded to energize in block 366a. However, the command signal given to each one specifies a different start delay time. Accordingly, there is a stagger between the actuation/closing of each finger.

In a second optional path 368, the microstimulators are consecutively energized by a delay Δ. Thus, microstimulator 1 (ST₁) is energized in block 368a, a delay is executed within the SCU 302 in block 368b, and so on for all of the microstimulators. Accordingly, paths 366 and 368 perform essentially the same function. However, in path 366 the interdevice timing is performed by the clocks within each implanted device 100 while in path 368, the SCU 302 is responsible for providing the interdevice timing.

In path 370, the SCU 302 actuates a first microstimulator (ST₁) in block 370a and waits in block 370b for its corresponding muscle to be actuated, as determined by microsensor 2 (SE₂), before actuating the remaining stimulators (ST₂-ST₅) in block 370c. This implementation could provide more coordinated movements in some situations.

Once the stimulators have been energized, as determined in block 364, closed loop grip pressure control is performed in blocks 372a and 372b by periodically reading the status of microsensor 3 (SE₃) and adjusting the commands given to the stimulators (ST₁-ST₅) accordingly. Consequently, this exemplary system has enabled the patient to regain control of his hand including coordinated motion and grip pressure control of the patient's fingers.

Referring again to **FIG. 3A**, a magnetic sensor 186 is shown. In the '284 patent, it was shown that such a sensor 186 could be used to disable the operation of an implanted device 100, e.g., to stop or otherwise alter the operation of such devices in an emergency situation, in response to a DC magnetic field, preferably from an externally positioned safety magnet 187 (see **FIG. 1**). Additionally, it is noted that power to at least some portions of a preferred implantable device may be removed when a magnetic field is sensed and thus power may be conserved. The magnetic sensor 186 can be implemented using various devices. Exemplary of such devices are devices manufactured by Nonvolatile Electronics, Inc. (e.g., their AA, AB, AC, AD, or AG series), Hall effect sensors, magnetoresistive sensors, and subminiature reed switches. Such miniature devices are configurable to be placed within the housing of the SCU 302 and implantable devices 100. While essentially passive magnetic sensors, e.g., reed switches, are possible, the remaining devices may include active circuitry that consumes power during detection of the DC magnetic field. Accordingly, it is preferred that controller 130 periodically, e.g., once a second, provide power to the magnetic sensor 186 and sample the magnetic sensor's output signal 374 during that sampling period. Additionally, a magnetoresistive sensor is especially preferred due to its small size that enables its use within the preferred implantable device 100 while conserving the available internal package volume.

The battery 104 used for powering the implantable device 100 (or SCU 302) is made from appropriate materials so as to preferably provide a power capacity of at least 1 microwatt-hour. Preferably, such a battery, e.g., a Li-I battery, has an energy density of about 240 mw-Hr/cm³. The battery voltage V of an exemplary battery is nominally 3.6 volts, which is more than adequate for operating the CMOS circuits preferably used to implement the IC chip(s) 216, and/or other electronic circuitry, within the SCU 302.

The battery 104 may take many forms, any of which may be used so long as the battery can be made to fit within the small volume available. The battery 104 may be either a primary battery or a rechargeable battery. A primary battery offers the advantage of not requiring a recharging circuit and the disadvantage of not being rechargeable (which means once its energy has been used up, the Implanted device no longer functions).

A preferred system for forming the environment for use of the present Invention is comprised of an implanted SCU 302 and a plurality of implanted devices 100, each of which contains its own rechargeable battery 144. As such, a patient is essentially independent of any external apparatus between battery chargings (which generally occur no more often than once an hour and preferably no more often than once every 24 hours). However, for some treatment regimens, it may be adequate to use a power supply analogous to that described In commonly assigned U.S. Pat. No. 5,324,316 (herein referred to as they '316 patent) that only provides power while an external AC magnetic field is being provided, e.g., from charger 118. Additionally, it may be desired, e.g., from a cost or flexibility standpoint, to implement the SCU 302 as an external device, e.g., within a watch-shaped housing that can be attached to a patient's wrist in a similar manner to the patient control unit 174.

The power consumption of the SCU 302 is primarily dependent upon the circuitry implementation, preferably CMOS, the circuitry complexity and the clock speed. For a simple system, a CMOS implemented state machine will be sufficient to provide the required capabilities of the programmable controller 308. However, for more complex systems, e.g., a system where an SCU 302 controls a large number of implanted devices 100 in a closed loop manner, a microcontroller may be required. As the complexity of such microcontrollers Increases (along with its transistor count), so does its power consumption. Accordingly, a larger battery having a capacity of 1 to 10 watt-hours is preferred. While a primary battery is possible, it is preferable that a rechargeable battery be used. Such larger batteries will require a larger volume and accordingly, cannot be placed in the injectable housing described above.

Since only one SCU is required to implement a system, the battery life of the SCU may be accommodated by Increasing the casing size (e.g., increasing at least one dimension to be in excess of 1 inch) for the SCU to accommodate a larger sized battery and either locating a larger SCU 302a (see **FIG. 1**) external to patient's body or a larger SCU 302b may be surgically Implanted.

Essentially, there have been described two classes of implantable devices 100, a first which is typically referred to as being RF powered, i.e., it does not contain a battery but instead receives all of its operating power from an externally-provided AC magnetic field (which field is preferably modulated to additionally wirelessly communicate commands to the implantable devices 100), and a second class which is referred to as battery powered which is powered by an internally provided battery which, in turn, is preferably rechargeable and periodically recharged by a similar externally-provided magnetic field (see, for example, commonly assigned U.S. Patent Application Publication No. 2003/0078634, which describes recharging environments and techniques for use with such implantable devices) but preferably receives its wireless commands via a modulated RF signal. Thus, in this case, the wireless command signal may be distinct from the wireless charging signal, However, In most other ways, these two classes of implantable devices are similar, e.g., they have similar size restrictions, are suitable for implantation via injection, and can similarly stimulate neural pathways and, thus, they are accordingly generally Interchangeable in embodiments of the present Invention its and environments. Alternatively, embodiments of the present invention may Include combinations of RF and battery-powered devices to take advantage of differences, e.g., cost and functional, between both classes of devices.

Accordingly, the use of the aforedescribed devices can be used to form a system that could restore motor functions to a patient with significant neurological damage, e.g., as a result of a significant, e.g., C2, spinal cord injury. Once motor functions are restored, the patient should also have their breathing functionality restored so that they can move without a ventilator. However, current Diaphragm Pacing Stimulation (DPS) systems provide fixed rate pacing and so such systems cannot increase the patient's ventilation rate to accommodate the needs of a patient with restored motor movement. This invention may also applicable to other medical conditions, known to exist in some young children, Amyotrophic Lateral Sclerosis (ALS), cancer or tumorous growths, complications from surgery, etc., where a patient is otherwise ventilator dependent but where the patient does have at least some motor movement.

There are various techniques available to determine the ventilation needs of a patient. In a first class of techniques, physical movements of the patient are sensed with an accelerometer or the like. Since a patient's movements have a strong correlation to the patient's current and future respiratory needs, this measurement can be used to determine the desired pacing rate for a DPS system. Since an accelerometer may show momentary peaks, e.g., impulse movements from movements/bumps of a vehicle that is carrying the patient, accelerometer measurements may not correspond to increases in physiological need. Thus, it is preferable that the output of an accelerometer be filtered, e.g., low pass filtered, to remove such a momentary increase. Such filtering may be done in either the analog domain, i.e., a hardware filter, or in the digital domain, e.g., via a software filter. Such filtering techniques are well known and need no further explanation here. Furthermore, since the onset of new movements sensed by an accelerometer will precede the patient's need for increased respiration, low pass filtering of the accelerometer's sensed output and responsively ramping up of the diaphragmatic pacing rate or the use of a time delay before increasing the diaphragmatic pacing rate will more closely match the patient's actual physiological need. Additionally, the use of multiple, e.g., multi-axis, accelerometers may be used to determine the nature of the movement and a weighted, e.g., hardware or software, average of its inputs may be used to determine if the sensed movement should effect the patient's respiration rate. Accordingly, in the referenced figures, a referenced accelerometer may also correspond to a plurality of accelerometers and may also include filtering circuitry or associated software (or such circuitry or associated software may be included within the controller 130 that responds to the accelerometers inputs). Exemplary embodiments of such single and multi-axis accelerometers are found in U.S. Pat. No. 6,466,821 (describing a multi-axis implementation) and U.S. Patent Application Publication No. 2004/0153127, U.S. Patent Application Publication No. 2004/0158294, and U.S. Patent Application Publication No. 2004/0183607.

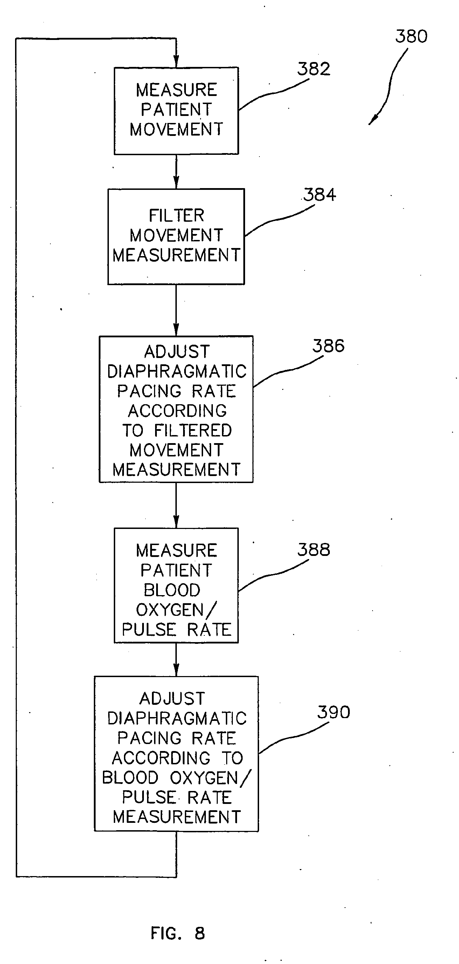

Alternatively or additionally, the use of an oximeter, e.g., a pulse oximeter, may be used as an indicator of current physiological need. Since the purpose of respiration is to provide oxygen to the patient's blood, a decrease in the oxygen level corresponds to a need to increase the respiration rate, i.e., in this case, a need to Increase the pacing rate. Similarly, such a device may also or alternatively be used to measure the patient's heart rate. Since, a patient's heart rate Increases with physiological need, embodiments of the present invention may use this data to determine if an increase In the diaphragmatic pacing rate is needed or to confirm that the resulting increase In the pacing rate corresponds to the Increase in heart rate. Embodiments of the present Invention may also include systems that measure movement via accelerometers or the like and oxygen level and/or heart rate sensors and process all of these parameters to determine the desired diaphragmatic pacing rate. In such systems, the output of the accelerometer (preferably with some filtering) may be used to respond to the patient's future and current respiration needs and the heart rate / oxygen level may be used to respond to the patient's long term respiration need or to the patient's current respiration deficit. This distinction is made In that the beginning of additional movements will anticipate the need for increasing respiration while heart rate / oxygen level will show or confirm whether the respiration rate increases have been met. Exemplary of such an oximeter is commonly assigned U.S. Patent Application Publication No. 2004/0210263 FIG. 8 shows a simplified exemplary procedure 380 for adjusting the patient's diaphragmatic pacing rate in response to the patient's measured physiological needs as has been discussed herein. Basically, the patient's movements are measured in block 382, e.g., by an accelerometer, filtered in block 384, and the patient's diaphragmatic pacing rate is adjusted accordingly in block 386. Optionally, a similar procedure occurs in blocks 388 and 390 where the patient's blood oxygen level and/or the patient's pulse rate is measured, e.g., by a pulse oximeter, and the patient's diaphragmatic pacing rate is adjusted accordingly. The process continuously repeats to allow the pacing rate to correspond to the measurements.

As will be shown below In exemplary embodiments of the present invention, such physiological need sensors may be wholly Internal to the patient's body, partially Internal to the patient's body (e.g., a portion of the sensor may be internal to the patient's body and another portion may be externally located or may have one or more sensors internally located while one or more sensors may be externally located), or may be wholly external to the patient's body. Similarly, the same position matrix is possible for systems of the present invention, i.e., the whole system may be internal, some portions may be internal with other portions external, or essentially the whole system may be external (with the exception of the stimulation leads which must be internally located). Furthermore, such systems may be internally powered, e.g., battery powered from an internal, preferably rechargeable, battery that is periodically recharged (preferably via an externally-provided AC magnetic field), or may be RF powered, i.e., rely upon an externally-provided AC magnetic field to supply power only when the AC magnetic field is present.

Furthermore, commonly assigned U.S. Pat. No. 6,738,672, referred to herein as the leaded BION® patents (note, BION® is a registered trademark of Advanced Bionics Corporation), are particularly relevant In that they describe techniques for attaching leads to the aforedescribed implantable devices that can then be connected, e.g., via lead splices when necessary, to leaded electrodes, e.g., Peterson Electrodes by Axon, Inc.

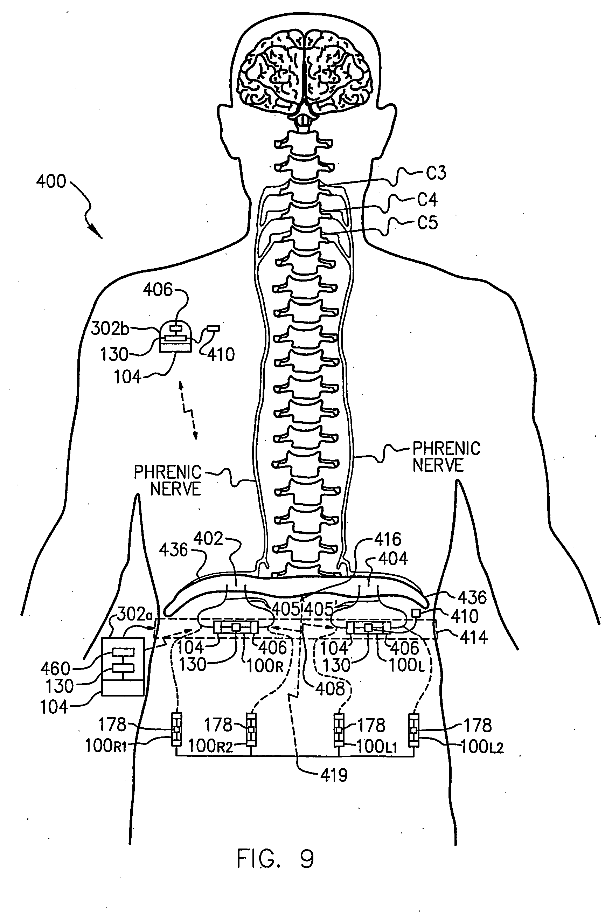

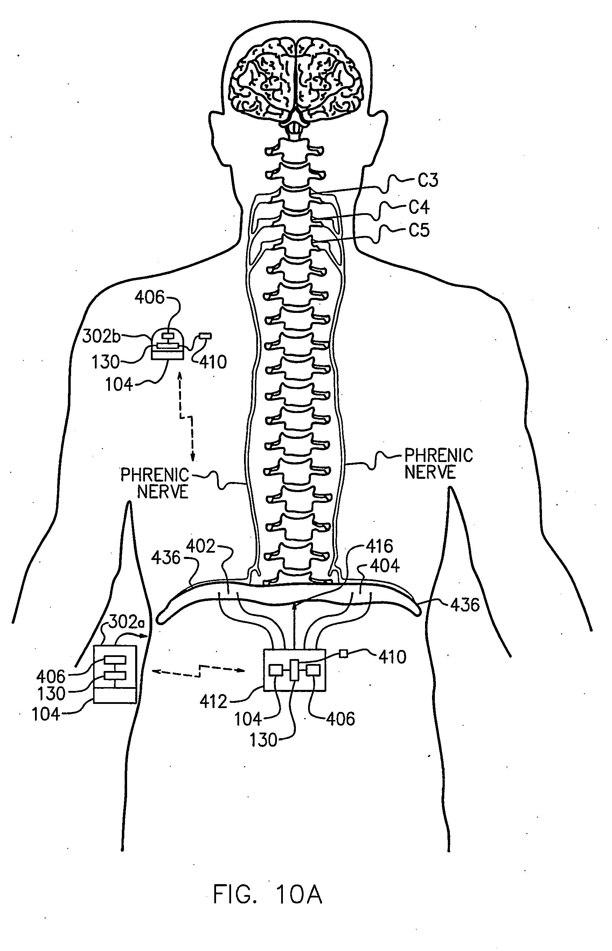

FIG. 9 shows a first exemplary embodiment 400 of the present invention. In FIG. 9, a pair of the aforedescribed implantable devices 100_{R} and 100_{L} (using the leaded BION® option) are coupled respectively to the right 402 and left 404 hemidiaphragm motor points, using Peterson electrodes 405, 405' or the like. Within at least one of these implantable devices 100 is an accelerometer 406 to measure patient movement (corresponding to physiological need). Accelerometer 406 Is coupled to the controller 130 and each of them preferably receive power from an Internal battery 104 (see FIG. 3A). Preferably, a communication link 408, e.g., an RF, modulated magnetic field, etc., is established between these devices to allow them to essentially synchronize their pulse generators to essentially concurrently stimulate the right 402 and left 404 hemidiaphragm motor points. Accordingly, the pacing rate for each of these implantable devices 100 is adjusted to correspond to the determined physiological need. Preferably, when each of the devices 100 has an accelerometer 406, data from each of the accelerometers 406 may be combined, e.g., averaged, to determine the actual physiological need. Alternatively, one accelerometer 406 may be held in reserve as a redundant sensor in case of a failure of the other accelerometer, e.g., a determination may be made from past history to determine if one of the accelerometers 406 is delivering an unrealistic value. Alternatively or additionally, an oximeter 410 (e.g., a pulse oximeter which optionally may alternatively or additionally detect the patient's pulse rate) may be connected to one or more of the implantable devices 100 (see, for example, implantable device 100_{L}) to provide an alternative or additional sensed value corresponding to physiological need. This data may be processed as has been previously discussed. To provide coupling to such an external device, coupling techniques may be used as described in commonly assigned U.S. patent application No. US 7450998 referred to herein as the'836 application. Alternatively, using the techniques described in the '836 application, a single implantable device may be used to combined the functionality of the two implantable devices 100_{R} and 100_{L} Into a single implantable device 412 having multiple stimulators and multiple outputs into a single device (see **FIG. 10A**).

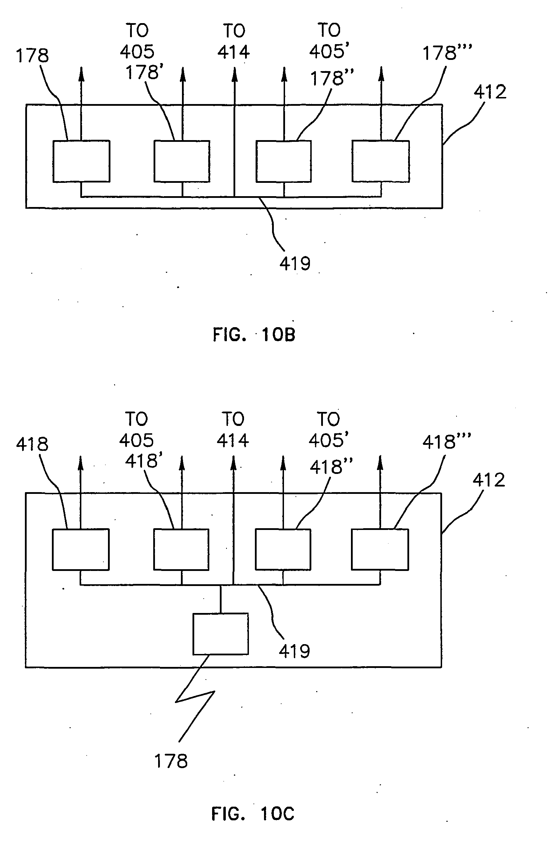

In this first configuration, stimulation pulses, preferably biphasic, are provided across right 402 and left 404 hemidiaphragm motor points using electrode pair 405, 405', e.g., preferably a bipolar electrode. Alternatively, two or more motor points may be individually stimulated on each of the two hemidiaphragms relative to a common indifferent electrode 416. Accordingly, an alternative embodiment is shown in **FIG. 9** where the right hemidiaphragm is driven by a pulse generator 178 (see **FIG. 1**) within each of a first and second implantable devices 100_{R1}and 100_{R2} and the left hemidiaphragm Is driven by a pulse generator 178 with each of a first and second implantable devices 100,_{L1} and 100_{L2}. Preferably, each of the pulse generators 178 is driven at essentially the same rate and phase, all relative to a common return 419. In **FIG.10****B.** essentially the same structure is used with the single device, a dual channel stimulator 412, with the difference being four pulse generators 178, 178', 178", and 178"' are all contained within the same device 412. **FIG. 10C** shows a next alternative configuration where a single pulse generator 178 is used to provide stimulation pulses through four discrete buffers 418, 418', 418", 418"' all relative to a common return 419.

In the embodiments described so far, the software to synchronize operation of the Implantable devices may be provided from a clinician's programmer 172, an external SCU 302a, or a patient control unit 174 (see **FIG. 1**). Alternatively, a master controller may be used to communicate with implantable devices 100_{R} and 100_{L}, e.g., via the communication protocol described In commonly assigned U.S. Pat. No. 6,472,991. The master controller may be either an implantable device, e.g., SCU 302b, or an external SCU 302a. Alternatively, either of these devices may contain an accelerometer 406 similarly coupled to controller 130 and battery 104 within the implantable SCU 302b and may be additionally coupled to an oximeter 410. ln such a case, the implantable devices 100_{R} and 100_{L} may be essentially slave devices and the master controller may make the determinations as to physiological need and pass commands to the implantable devices 100_{R} and 100_{L} to control the stimulation rate of the diaphragm accordingly. Notably, when an SCU 302 is used, this SCU 302 may also communicate with a plurality of other devices (see, for example, the implantable devices 100 of FIG. 1) which may also restore motor functions and movements to the patient as previously described.

Preferably, implantable devices 100_{R} and 100_{L} are battery powered, e.g., battery 104 is contained within and periodically recharged via an external charger 118 or the like and may be controlled from either an internal SCU 302b or external SCU 302a. Alternatively, implantable devices 100_{R} and 100_{L} are RF powered and receive power and/or commands, e.g., via a modulated AC magnetic field provided via a coil 414 as shown in **FIG. 9** (see, for example, U.S. patent application Publication 2003/0234631).

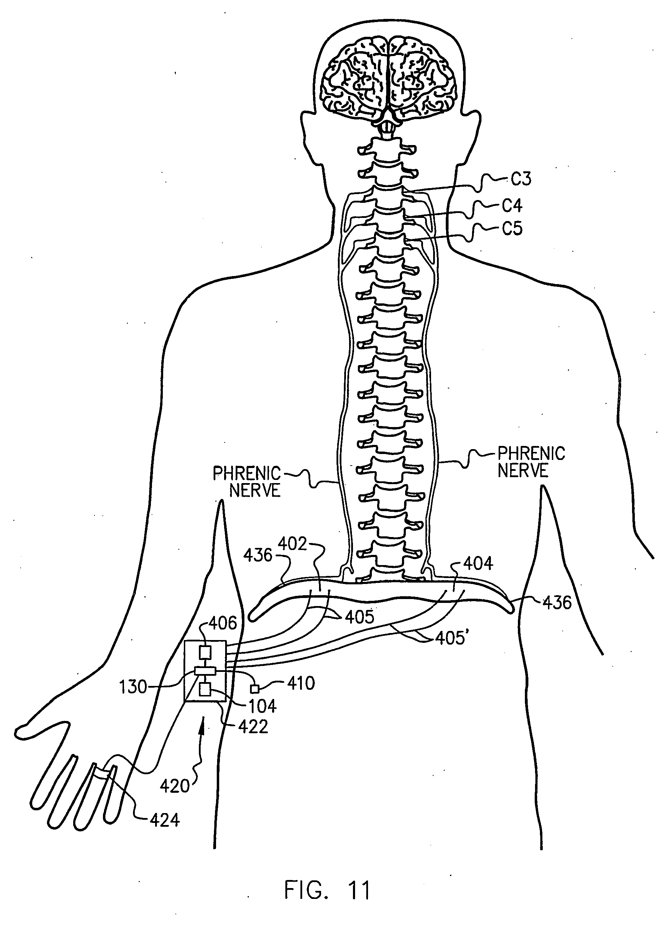

**FIG. 11** shows an alternative exemplary embodiment 420 in which an external DPS stimulator 422 is percutaneously attached, e.g., via an epigastric port, to internal electrodes 405, 405' at the right 402 and left 404 hemidiaphragm motor points. Preferably in this embodiment, accelerometer 406 is contained within external DPS stimulator 422 and data measured by this accelerometer 406 is used to control the diaphragm stimulation rate. Alternatively or additionally, a finger cuff pulse oximeter 424 may be coupled to the DPS stimulator 422 or percutaneously to oximeter 410 and this data may be used in the algorithm for determining the diaphragm pacing rate.

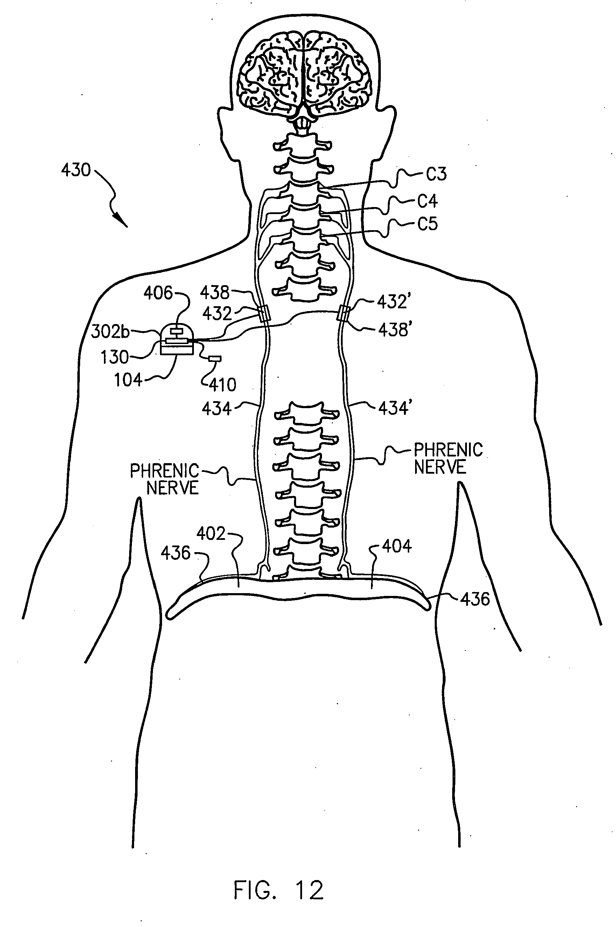

Accordingly, what has been shown is a system that provides adjustable diaphragmatic pacing to a patient having an associated neurological deficit wherein the adjustments automatically occur In response to the patient's physiological need, While the invention has been described by means of specific embodiments and applications thereof, it is understood that numerous modifications and variations could be made thereto by those skilled in the art. For example, while the prior discussion has been addressed to a DPS system that stimulates the motor points of the right and left hemidiaphragms, the use of electrodes 432, 432' inserted into or around, e.g., using cuff electrodes, the phrenic nerves 434, 434' (which In turn stimulates the right 402 and left 404 hemidiaphragm motor points of the patient's diaphragm 436) is also considered to be an embodiment (see exemplary embodiment 430 in **FIG. 12**) within the scope of the present invention. Additionally, in this embodiment, at least a portion of case 433 of SCU 302b is conductive and serves as an indifferent, return electrode for electrodes 432, 432'. Alternatively, as described In the previously incorporated '836 application, a stimulator / cuff electrode combination 438, 438' may be used that clamps to each of the phrenic nerves 434, 434'. Since the lengths of the phrenic nerves differ, a time lag is preferably programmed between the stimulation pulses emitted from each of the electrodes 432, 432' or the stimulator / cuff electrode combinations 438, 438'. Other features may also be present in embodiments of the present invention. As has been discussed above, implantable devices 100 may sense as well as stimulate. Such devices may alternate between stimulating and sensing and thus may confirm that muscles in the diaphragm have depolarized following a stimulation pulse, and if not, the stimulation parameters may automatically be readjusted to assure the depolarization occurs. Finally, it may also be desirable to periodically have the stimulation parameters readjust to allow for a periodic deep breath. Accordingly, such variations may be made without departing from the spirit and scope of the invention. It is therefore to be understood that within the scope of the claims, the invention may be practiced otherwise than as specifically described herein.

## Claims

1. A system (300) for controlling respiration in a patient having an associated neurological deficit by using an electronic device (100) configured for adjustably diaphragmatically pacing in response to the patient's physiological need, said system (300) comprising:
one or more pulse generators (178) configured for delivering stimulation pulses and coupling to a plurality of electrodes (405) adapted to be placed proximate to the left and right hemidiaphragm motor points that respond to said stimulation pulses to induce respiration, wherein said one or more pulse generators (178) operate at a stimulation rate;
a physiological need sensor for measuring a parameter corresponding to the patient's physiological need and configured for measuring the patient's activity level , wherein said stimulation rate of said one or more pulse generators (178) is periodically adjusted according to said parameter measured by said physiological need sensor

2. The system (300) of claim 1 wherein said physiological need sensor comprises an oximeter (410) suitable for implantation within the patient's body.

3. The system (300) of claim 1 wherein said physiological need sensor comprises an oximeter (410) suitable for use external to the patient's body.

4. The system (300) of claim 1 wherein said physiological need sensor configured for measuring the patient's activity level comprises one or more accelerometers (406).

5. The system (300) of claim 4 wherein said physiological need sensor additionally comprises an oximeter (410).

6. The system (300) of claim 1 wherein said physiological need sensor comprises two or more accelerometers (406) oriented at approximately right angles to each other; and wherein
said stimulation rate is periodically adjusted according to weighted measurements from said two or more accelerometers (406).

7. The system (300) of claim 1 wherein said one or more pulse generators (178) are configured for placement external to the patient's body for coupling to electrodes (405) within the patient's body.

8. The system (300) of claim 1 wherein said one or more pulse generators (178) are configured for implantation within the patient's body.

9. The system (300) of claim 1 wherein said sensor and at least one of said pulse generators (178) are contained within a sealed elongate housing (206) having an axial dimension of less than 60 mm and a lateral dimension of less than 6 mm that is suitable for implantation within the patient's body.

10. The system (300) of claim 1 comprising at least two implantable devices (100) each contained within a sealed elongate housing (206) having an axial dimension of less than 60 mm and a lateral dimension of less than 6 mm wherein each of said implantable devices (100) has at least one pulse generator (178) contained within and at least one of said implantable devices (100) contains said sensor for measuring a parameter corresponding to the patient's physiological need and wherein said implantable devices (100) communicate with each other and thereby coordinate operations of each of their respective pulse generators (178),

11. The system (300) of claim 9 additionally comprising:
a master controller (302a, 302b) in periodic wireless communication with said one or more pulse generators (178) and said physiological need sensor; and wherein
said master controller (302a, 302b) periodically calculates a new desired stimulation rate for said one or more pulse generators (178) in response to said measured parameter from said physiological need sensor and communicates said new desired stimulation rate to said one or more pulse generators (178).

12. The system (300) of claim 11 wherein said master controller (302b) is suitable for implantation within the patient's body.

13. The system (300) of claim 11 wherein said master controller (302a) is configured for use external to the patient's body.

14. The system (300) of claim 11 wherein said master controller (302a, 302b) additionally comprises said sensor for measuring a parameter corresponding to the patient's physiological need.

15. The system (300) of claim 11 wherein said sensor is suitable for placement external to the patient's body.

16. The system (300) of claim 1 additionally comprising:
a master controller (302) in periodic wireless communication with said one or more pulse generators (178) and said physiological need sensor; and wherein
said master controller (302) periodically calculates a new desired stimulation rate for said one or more pulse generators (178) in response to said measured parameter from said physiological need sensor and communicating said new desired stimulation rate to said one or more pulse generators (178).

17. The system (300) of claim 1 additionally comprising:
a master controller (302);
a plurality of implantable devices (100) suitable for stimulation of neurological pathways, wherein each of said implantable devices (100) is contained within a sealed elongate housing (206) having an axial dimension of less than 60 mm and a lateral dimension of less than 6 mm and is under control of wireless signals from said master controller (302); and wherein
said one or more pulse generators (178) are implantable and under control of said master controller (302).

18. The system (300) of claim 17 wherein said master controller (302) is implantable.

19. The system (300) of claim 1 additionally comprising a power source (316) for supplying power to said one or more pulse generators (178) and said sensor.

20. The system (300) of claim 19 wherein said power source (316) comprises a rechargeable battery (104) configured for receiving recharging power from an externally-provided alternating magnetic field.

21. The system (300) of claim 19 wherein said power source (316) comprises a capacitor for receiving power from an externally-provided alternating magnetic field.

22. The system (300) of claim 1 wherein said electrodes are proximate to a neurological pathway that comprises the patient's phrenic nerves.

23. The system (300) of claim 1 wherein said physiological need sensor comprises a pulse oximeter (410,424).

## Patentansprüche

1. System (300) zur Steuerung der Atmung bei einem Patienten mit einer neurologischen Schädigung unter Verwendung einer elektronischen Vorrichtung (100), die ausgebildet ist, um anpassbar in Reaktion auf die physiologischen Bedürfnisse des Patienten als Zwerchfellschrittmacher zu dienen, wobei das System (300) Folgendes umfasst:
einen oder mehrere Impulsgeneratoren (178), die ausgebildet sind um Stimulationsimpulse abzugeben und an eine Vielzahl von Elektroden (405) angeschlossen zu werden, die geeignet sind, um nahe der motorischen Punkte der linken und rechten Zwerchfellhälfte (Hemidiaphragma) angeordnet zu werden, die auf die Stimulationsimpulse reagieren, um Atmung zu induzieren, wobei der eine oder die mehreren Impulsgeneratoren (178) in einer Stimulationsrate betrieben werden;
einen Sensor für physiologische Bedürfnisse zur Messung eines Parameters, der den physiologischen Bedürfnissen des Patienten entspricht, wobei der Sensor ausgebildet ist, um das Ausmaß der Aktivität des Patienten zu messen, wobei die Stimulationsrate des einen oder der mehreren Impulsgeneratoren (178) regelmäßig gemäß dem durch den Sensor für physiologische Bedürfnisse gemessenen Parameter angepasst wird.

2. System (300) nach Anspruch 1, worin der Sensor für physiologische Bedürfnisse ein Oximeter (410) umfasst, das geeignet ist, um in den Körper des Patienten implantiert zu werden.

3. System (300) nach Anspruch 1, worin der Sensor für physiologische Bedürfnisse ein Oximeter (410) umfasst, das zur Anwendung außerhalb des Körpers des Patienten geeignet ist.

4. System (300) nach Anspruch 1, worin der Sensor für physiologische Bedürfnisse, der zur Messung des Ausmaßes der Aktivität eines Patienten ausgebildet ist, einen oder mehrere Beschleunigungsmesser (406) umfasst.

5. System (300) nach Anspruch 4, worin der Sensor für physiologische Bedürfnisse zusätzlich ein Oximeter (410) umfasst.

6. System (300) nach Anspruch 1, worin der Sensor für physiologische Bedürfnisse zwei oder mehrere Beschleunigungsmesser (406) umfasst, die etwa im rechten Winkel in Bezug aufeinander ausgerichtet sind, und worin die Stimulationsrate regelmäßig gemäß gewichteten Messungen der zwei oder mehreren Beschleunigungsmesser (406) angepasst wird.

7. System (300) nach Anspruch 1, worin der eine oder die mehreren Impulsgeneratoren (178) ausgebildet sind, um außerhalb des Körpers des Patienten angeordnet zu werden, um an Elektroden (405) im Inneren des Körpers des Patienten angeschlossen zu werden.

8. System (300) nach Anspruch 1, worin der eine oder die mehreren Impulsgeneratoren (178) zur Implantation in den Körper des Patienten ausgebildet sind.

9. System (300) nach Anspruch 1, worin der Sensor und zumindest einer der Impulsgeneratoren (178) in einem abgedichteten länglichen Gehäuse (206) mit einer axialen Ausdehnung von weniger als 60 mm und einer lateralen Ausdehnung von weniger als 6 mm enthalten sind, das zur Implantation in den Körper des Patienten geeignet ist.

10. System (300) nach Anspruch 1, das Folgendes umfasst: zumindest zwei implantierbare Vorrichtungen (100), die jeweils in einem abgedichteten länglichen Gehäuse (206) mit einer axialen Ausdehnung von weniger als 60 mm und einer lateralen Ausdehnung von weniger als 6 mm enthalten sind, wobei jede der implantierbaren Vorrichtungen (100) zumindest einen darin enthaltenen Impulsgenerator (178) aufweist, und wobei zumindest eine der implantierbaren Vorrichtungen (100) den Sensor zur Messung eines Parameters umfasst, der den physiologischen Bedürfnissen des Patienten entspricht, und worin die implantierbaren Vorrichtungen (100) miteinander kommunizieren und dadurch den Betrieb ihres jeweiligen Impulsgenerators (178) miteinander abstimmen.

11. System (300) nach Anspruch 9, das zusätzlich dazu Folgendes umfasst:
eine Hauptsteuervorrichtung (302a, 302b), die in periodischer drahtloser Kommunikation mit dem einen oder den mehreren Impulsgeneratoren (178) und dem Sensor für physiologische Bedürfnisse steht, und worin
die Hauptsteuervorrichtung (302a, 302b) in Reaktion auf den durch den Sensor für physiologische Bedürfnisse gemessenen Parameter periodisch eine neue gewünschte Stimulationsrate für den einen oder die mehreren Impulsgeneratoren (178) berechnet und die neue gewünschte Stimulationsrate an den einen oder die mehreren Impulsgeneratoren (178) kommuniziert.

12. System (300) nach Anspruch 11, worin die Hauptsteuervorrichtung (302b) zur Implantation in den Körper des Patienten geeignet ist.

13. System (300) nach Anspruch 11, worin die Hauptsteuervorrichtung (302a) für die Verwendung außerhalb des Körpers des Patienten ausgebildet ist.

14. System (300) nach Anspruch 11, worin die Hauptsteuervorrichtung (302a, 302b) zusätzlich den Sensor zur Messung eines Parameters, der den physiologischen Bedürfnissen des Patienten entspricht, umfasst.

15. System (300) nach Anspruch 11, worin der Sensor zur Anordnung außerhalb des Körpers des Patienten geeignet ist.

16. System (300) nach Anspruch 1, das zusätzlich Folgendes umfasst:
eine Hauptsteuervorrichtung (302) in periodischer drahtloser Kommunikation mit dem einen oder den mehreren Impulsgeneratoren (178) und dem Sensor für physiologische Bedürfnisse und worin
die Hauptsteuervorrichtung (302) in Reaktion auf den durch den Sensor für physiologische Bedürfnisse gemessenen Parameter periodisch eine neue gewünschte Stimulationsrate für den einen oder die mehreren Impulsgeneratoren (178) berechnet und die neue gewünschte Stimulationsrate an den einen oder die mehreren Impulsgeneratoren (178) kommuniziert.

17. System (300) nach Anspruch 1, das zusätzlich Folgendes umfasst:
eine Hauptsteuervorrichtung (302);
eine Vielzahl implantierbarer Vorrichtungen (100), die zur Stimulation neurologischer Signalwege geeignet ist, wobei jede der implantierbaren Vorrichtungen (100) in einem abgedichteten länglichen Gehäuse (206) mit einer axialen Ausdehnung von weniger als 60 mm und einer lateralen Ausdehnung von weniger als 6 mm enthalten ist und durch drahtlos übertragene Signale von der Hauptsteuervorrichtung (302) gesteuert wird, und worin
der eine oder die mehreren Impulsgeneratoren (178) implantierbar sind und durch die Hauptsteuervorrichtung (302) gesteuert werden.

18. System (300) nach Anspruch 17, worin die Hauptsteuervorrichtung (302) implantierbar ist.

19. System (300) nach Anspruch 1, das zusätzlich eine Stromquelle (316) zur Zufuhr von Strom an den einen oder die mehreren Impulsgeneratoren (178) und den Sensor umfasst.

20. System (300) nach Anspruch 19, worin die Stromquelle (316) eine wiederaufladbare Batterie (104) umfasst, die ausgebildet ist, um Ladestrom von einem extern bereitgestellten, magnetischen Wechselfeld zu beziehen.

21. System (300) nach Anspruch 19, worin die Stromquelle (316) einen Kondensator umfasst, um Strom von einem extern bereitgestellten, magnetischen Wechselfeld zu beziehen.

22. System (300) nach Anspruch 1, worin die Elektroden in der Nähe von einem neurologischen Signalpfad vorliegen, der die Zwerchfellsnerven des Patienten umfasst.

23. System (300) nach Anspruch 1, worin der Sensor für physiologische Bedürfnisse ein Pulsoximeter (410, 424) umfasst.

## Revendications

1. Système (300) pour commander la respiration d'un patient présentant un déficit neurologique associé en utilisant un dispositif électronique (100) configuré pour l'électrostimulation de manière ajustable du diaphragme en réponse au besoin physiologique du patient, ledit système (300) comprenant :
un ou plusieurs générateurs d'impulsion (178) configurés pour délivrer des impulsions de stimulation et pour un couplage à une pluralité d'électrodes (405) conçues pour être placées à proximité des points moteurs des hémi-diaphragmes gauche et droit qui répondent auxdites impulsions de stimulation pour induire une respiration, dans lequel lesdits un ou plusieurs générateurs d'impulsion (178) fonctionnent à une fréquence de stimulation ;
un capteur de besoin physiologique pour mesurer un paramètre correspondant au besoin physiologique du patient et configuré pour mesurer le niveau d'activité du patient, dans lequel ladite fréquence de stimulation desdits un ou plusieurs générateurs d'impulsion (178) est ajustée périodiquement en fonction dudit paramètre mesuré par ledit capteur de besoin physiologique.

2. Système (300) selon la revendication 1, dans lequel ledit capteur de besoin physiologique comprend un oxymètre (410) approprié pour une implantation dans le corps du patient.

3. Système (300) selon la revendication 1, dans lequel ledit capteur de besoin physiologique comprend un oxymètre (410) approprié pour une utilisation à l'extérieur du corps du patient.

4. Système (300) selon la revendication 1, dans lequel ledit capteur de besoin physiologique configuré pour mesurer le niveau d'activité du patient comprend un ou plusieurs accéléromètres (406).

5. Système (300) selon la revendication 4, dans lequel ledit capteur de besoin physiologique comprend en plus un oxymètre (410).

6. Système (300) selon la revendication 1, dans lequel ledit capteur de besoin physiologique comprend deux accéléromètres (406) ou plus orientés à peu près à angle droit les uns par rapport aux autres ; et dans lequel
ladite fréquence de stimulation est ajustée périodiquement en fonction de mesures pondérées provenant desdits deux accéléromètres (406) ou plus.

7. Système (300) selon la revendication 1, dans lequel lesdits un ou plusieurs générateurs d'impulsion (178) sont configurés pour être placés à l'extérieur du corps du patient pour un couplage à des électrodes (405) dans le corps du patient.

8. Système (300) selon la revendication 1, dans lequel lesdits un ou plusieurs générateurs d'impulsion (178) sont configurés pour une implantation dans le corps du patient.

9. Système (300) selon la revendication 1, dans lequel ledit capteur et au moins l'un desdits générateurs d'impulsion (178) sont contenus dans un logement allongé fermé hermétiquement (206) ayant une dimension axiale inférieure à 60 mm et une dimension latérale inférieure à 6 mm qui est approprié pour une implantation dans le corps du patient.

10. Système (300) selon la revendication 1, comprenant au moins deux dispositifs implantables (100) contenus chacun dans un logement allongé fermé hermétiquement (206) ayant une dimension axiale inférieure à 60 mm et une dimension latérale inférieure à 6 mm, dans lequel chacun desdits dispositifs implantables (100) a au moins un générateur d'impulsion (178) contenu dans celui-ci et au moins l'un desdits dispositifs implantables (100) contient ledit capteur pour mesurer un paramètre correspondant au besoin physiologique du patient, et dans lequel lesdits dispositifs implantables (100) communiquent les uns avec les autres et coordonnent de ce fait les fonctionnements de chacun de leurs générateurs d'impulsion (178) respectifs.

11. Système (300) selon la revendication 9, comprenant en plus :
un contrôleur maître (302a, 302b) en communication sans fil périodique avec lesdits un ou plusieurs générateurs d'impulsion (178) et ledit capteur de besoin physiologique ; et dans lequel
ledit contrôleur maître (302a, 302b) calcule périodiquement une nouvelle fréquence de stimulation souhaitée pour lesdits un ou plusieurs générateurs d'impulsion (178) en réponse au dit paramètre mesuré provenant dudit capteur de besoin physiologique et communique ladite nouvelle fréquence de stimulation souhaitée auxdits un ou plusieurs générateurs d'impulsion (178).

12. Système (300) selon la revendication 11, dans lequel ledit contrôleur maître (302b) est approprié pour une implantation dans le corps du patient.

13. Système (300) selon la revendication 11, dans lequel ledit contrôleur maître (302a) est configuré pour une utilisation à l'extérieur du corps du patient.

14. Système (300) selon la revendication 11, dans lequel ledit contrôleur maître (302a, 302b) comprend en plus ledit capteur pour mesurer un paramètre correspondant au besoin physiologique du patient.

15. Système (300) selon la revendication 11, dans lequel ledit capteur est approprié pour être placé à l'extérieur du corps du patient.

16. Système (300) selon la revendication 1, comprenant en plus :
un contrôleur maître (302) en communication sans fil périodique avec lesdits un ou plusieurs générateurs d'impulsion (178) et ledit capteur de besoin physiologique ; et dans lequel
ledit contrôleur maître (302) calcule périodiquement une nouvelle fréquence de stimulation souhaitée pour lesdits un ou plusieurs générateurs d'impulsion (178) en réponse au dit paramètre mesuré provenant dudit capteur de besoin physiologique et communique ladite nouvelle fréquence de stimulation souhaitée auxdits un ou plusieurs générateurs d'impulsion (178).

17. Système (300) selon la revendication 1, comprenant en plus :
un contrôleur maître (302) ;
une pluralité de dispositifs implantables (100) appropriés pour la stimulation de trajets neurologiques, dans lequel chacun desdits dispositifs implantables (100) est contenu dans un logement allongé fermé hermétiquement (206) ayant une dimension axiale inférieure à 60 mm et une dimension latérale inférieure à 6 mm et est sous la commande de signaux sans fil provenant dudit contrôleur maître (302) ; et dans lequel
lesdits un ou plusieurs générateurs d'impulsion (178) sont implantables et sont sous la commande dudit contrôleur maître (302).

18. Système (300) selon la revendication 17, dans lequel ledit contrôleur maître (302) est implantable.

19. Système (300) selon la revendication 1, comprenant en outre une source d'alimentation (316) pour alimenter lesdits un ou plusieurs générateurs d'impulsion (178) et ledit capteur.

20. Système (300) selon la revendication 19, dans lequel ladite source d'alimentation (316) comprend une batterie rechargeable (104) configurée pour recevoir une puissance de recharge d'un champ magnétique alternatif fourni de l'extérieur.

21. Système (300) selon la revendication 19, dans lequel ladite source d'alimentation (316) comprend un condensateur pour recevoir une puissance d'un champ magnétique alternatif fourni de l'extérieur.

22. Système (300) selon la revendication 1, dans lequel lesdites électrodes sont à proximité d'un trajet neurologique qui comprend les nerfs phréniques du patient.

23. Système (300) selon la revendication 1, dans lequel ledit capteur de besoin physiologique comprend un sphygmo-oxymètre (410, 424).
